# EUROPÄISCHE PATENTSCHRIFT

(11) **EP 1 073 412 B1**
(45) Veröffentlichungstag und Bekanntmachung des Hinweises auf die Patenterteilung: **28.11.2001**
(21) Anmeldenummer: 99923474.3
(22) Anmeldetag: 28.04.1999
(51) Int. Cl.: A61K 7/48, A61K 31/20, A61K 31/355

(54) **WIRKSTOFFKOMBINATIONEN AUS VITAMIN A- DERIVATE UND UBI- ODER PLASTOCHINONEN ENTHALTENDE HAUTPFLEGMITTEL**
SKIN CARE AGENTS CONTAINING COMBINATIONS OF ACTIVE AGENTS CONSISTING OF VITAMIN A DERIVATIVES AND UBI- OR PLASTOQUINONES
AGENTS DE SOINS POUR LA PEAU CONTENANT DES COMBINAISONS DE PRINCIPES ACTIFS A BASE DE DERIVES DE VITAMINE A ET D'UBIQUINONES OU DE PLASTOQUINONES

(30) Priorität: 07.05.1998 DE 19820392
(43) Veröffentlichungstag der Anmeldung: 07.02.2001
(73) Patentinhaber: GS DEVELOPMENT AB, 213 75 Malmö (SE)
(72) Erfinder: HOPPE, Udo, D-24598 Heidmühlen (DE); SCHREINER, Volker, D-20259 Hamburg (DE); STÄB, Franz, D-21379 Echem (DE)
(74) Vertreter: UEXKÜLL & STOLBERG
(86) Internationale Anmeldenummer: EP9902854
(87) Internationale Veröffentlichungsnummer: WO9956719

(56) Entgegenhaltungen:
- WO-A-95/11015
- WO-A-95/26180
- US-A- 5 362 494
- US-A- 5 378 461

## Beschreibung

Die Erfindung betrifft topische Zubereitungen mit Ubichinonen und/oder Plastochinonen.

Die Haut altert bedingt durch endogene, genetisch determinierte Einflüsse. Exogene Faktoren, wie UV-Licht und chemische Noxen, können kumulativ wirksam sein und beschleunigen die natürlichen Alterungsprozesse. Es kommt zu zahlreichen degenerativen Prozessen, die je nach Größe der Einflußfaktoren u.a. zu folgenden strukturellen Veränderungen und Schäden in Dermis und Epidermis führen (Dermatoheliosis):
a) Rückbildung des mikrovasculären Systems.
b) Schlaffheit und Ausbildung von Falten teilweise aufgrund einer Abnahme und Quervemetzung des Kollagens, Akkumulation der Glucosaminoglycane (Grundsubstanz).
c) Abflachung der Retezapfen. Damit verbunden ist die Verringerung der Fäche zwischen Dermis und Epidermis, über die Stoffe zur Ernährung und Entschlackung der Epidermis ausgetauscht werden.
d) Eingeschränkter regenerativer Turnover in der Epidermis, verbunden mit fehlerhafter Ausbildung der Hornschicht (Verhornungsstärungen), die zur Hautaustrocknung führt.
e) Fehlerhafte Regulation von Zellteilung (Proliferation) und Zellreifung (Differenzierung) in der Epidermis, woraus zelluläre Atypien und der Verlust der Polarität resultieren.
f) Lokale Hyper-, Hypo- und Fehlpigmentierungen (Altersflecken).

Bei der Pathogenese von Akne, die besonders im jugendlichen Alter, aber auch in späteren Jahren auftritt, spielen sowohl die Überproduktion von Talgdrüsenlipiden durch überentwickelte Talgdrüsen (Talgdrüsenhyperplasie) als auch Verhornungsstörungen des Talgdrüsenausganges eine wesentliche Rolle. In beiden Fällen kommt es zu einer Anreicherung von Sebumlipiden, die den Nährboden für Akneauslösende Bakterien bieten.

Für die Pigmentierung der Haut verantwortlich sind z.B. die Melanozyten, welche in der untersten Schicht der Epidermis, dem Stratum basale, neben den Basalzellen als - je nach Hauttyp, dem Stratum basale, neben den Basatzellen als - je nach Hauttyp entweder vereinzelt oder aber mehr oder weniger gehäuft auftretende pigmentbildende Zellen vorzufinden sind. Melanozyten enthalten als charakteristische Zellorganellen Melanosomen, die bei Anregung durch UV-Strahlung verstärkt Melanin bilden. Dieses wird in die Hornzellen (Keratinozyten) transportiert und ruft eine mehr oder weniger ausgeprägte bräunliche oder braune Hautfarbe hervor.

Melanin wird als Endstufe eines oxidativen Prozesses gebildet, in welchem Tyrosin unter Mitwirkung des Enzymes Tyrosinase über 3,4-Dihydroxyphenyl-2-Aminopropionsäure (Dopa), Dopa-Chinon, Leucodopachrom, Dopachrom, 5,6-Dihydroxyindol und Indol-5,6-chinon schließlich in Melanin umgewandelt wird.

Probleme mit Hyperpigmentierung der Haut haben vielfältige Ursachen bzw. sind Begleiterscheinungen vieler biologischer Vorgänge, z.B. UV-Strahlung (z.B. Sommersprossen, Ephelides), genetische Disposition, Fehlpigmentierung der Haut bei der Wundheilung bzw. -vernarbung oder der Hautalterung (z.B. Lentigines seniles).

Es sind Wirkstoffe und Zubereitungen bekannt, welche der Hautpigmentierung entgegenwirken. Im praktischen Gebrauch sind im wesentlichen Präparate auf der Grundlage von Hydrochinon. welche aber einesteils erst nach mehrwöchiger Anwendung ihre Wirkung zeigen, deren sehr lange Anwendung andererseits aus toxikologischen Gründen nicht immer unbedenklich ist. Auch die Inhibierung der Tyrosinase mit Substanzen wie Kojisäure, Ascorbinsäure und Azelainsäure sowie deren Derivaten ist zwar geläufig, hat aber kosmetische und dermatologische Nachteile.

Die vorliegende Erfindung betrifft insbesondere Produkte zur Pflege und Prophylaxe lichtgealterter bzw. zur Ausbildung von Akne neigender Haut sowie zur Behandlung insbesondere der unter a) - f) aufgeführten Folgeschäden der Lichtalterung und zur insbesondere kosmetischen Aufhellung ("Skin Whitening") von unerwünschter Pigmentierung der Haut.

Produkte zur Pflege, Prophylaxe und Behandlung lichtgealterter Haut sowie zur prophylaktischen Behandlung von Akne sind an sich bekannt. Sie enthalten z.B. Vitamin A-Säure. Ihre Wirkung auf die Strukturschäden bei Lichtalterung bzw. zur Minderung des Risikos der Akneentstehung ist allerdings umfangmäßig begrenzt. Die Verwendung Vitamin A-Säure-haltiger Produkte bedingt darüberhinaus oft starke erythematöse Hautreizungen. Insbesondere Vitamin A-Säure ist daher nur in geringen Konzentrationen einsetzbar.

Weiterhin sind kosmetische Zubereitungen mit Coenzym Q-10 aus der DE-A-33 09 850 bekannt, die zur Behandlung von Hautkrankheiten, zur Prophylaxe von dystrophischen und dysmetabolischen Zuständen der Haut und zur Anwendung bei chemischen und physikalischen Respirationsschäden oder bei verzögerter Respiration verbunden mit Alter und Abnutzung geeignet sind.

In der japanischen Offenlegungsschrift 58,180,410 ist die Eignung von Coenzym Q-10 für Kosmetika beschrieben. Es soll den Hautzellmetabolismus aktivieren und die Oxidation unterdrücken. Coenzym Q-10 hat im Resultat eine wichtige Funktion bei der Prävention von Hautschäden durch UV-Strahlen und der Prävention von Hautalterung. Bei 20 bis 40jährigen wird die Hautrauhigkeit gebessert, indem der Haut Feuchtigkeit gegeben wird.

Aus der WO95/26180 sind topische Zubereitungen bekannt, die Retinole, Retinale, beta-Carotin und Ubichinone oder Plastochinone enthalten.

Ziel der vorliegenden Erfindung war es somit, Wege zu finden, die Nachteile des Standes der Technik zu vermeiden. Insbesondere sollte die prophylaktische Wirkung bei Akne und die restrukturierende Wirkung bei Lichtalterung dauerhaft, nachteilig und ohne das Risiko von Nebenwirkungen sein.

Erfindungsgemäß werden diese Ziele von Hautpflegepräparaten erreicht, in denen Vitamin A-Säure oder deren Derivate in Kombination mit Ubichinonen und/oder Plastochinonen enthalten sind.

Gegenstand der Erfindung sind topische Zubereitungen mit einem Gehalt an einer Verbindung und mehreren Verbindungen, ausgewählt aus der Gruppe (A), bestehend aus Vitamin A-Säure oder deren Derivaten, in Kombination mit einem Gehalt an einer Verbindung oder mehreren Verbindungen, ausgewählt aus der Gruppe (B), bestehend aus Ubichinonen und deren Derivaten und Plastochinonen und deren Derivaten.

Bevorzugt werden Zubereitungen mit einem Gehalt an einer Verbindung oder zwei Verbindungen oder drei Verbindungen, ausgewählt aus der Gruppe (A), kombiniert mit einer Verbindung oder zwei Verbindungen oder drei Verbindungen aus der Gruppe (B).

Die erfindungsgemäßen topischen Zubereitungen können kosmetische oder dermatologische Zubereitungen sein, und sie werden hier auch als Hautpflegeprodukte oder Dermatika bezeichnet. Sie dienen, wie auch die Wirkstoffe, zur Prophylaxe bei Akne und zur Pflege und Prophylaxe bei Lichtalterung und zur Behandlung lichtgealterter Haut und zur Aufhellung unerwünschter Pigmentierung der Haut.

Gegenstand der Erfindung ist auch die Verwendung topischer Zubereitungen mit einem Gehalt an einer Verbindung oder mehreren Verbindungen ausgewählt aus der Gruppe (A), bestehend aus Vitamin A-Säure oder deren Derivaten, in Kombination mit einem Gehalt an einer Verbindung oder mehreren Verbindungen, ausgewählt aus der Gruppe (B), bestehend aus Ubichinonen und deren Derivaten und Plastochinonen und deren Derivaten, zur Pflege und prophylaktischen Behandlung bei Akne und zur Pflege und Prophylaxe bei Lichtalterung und zur Behandlung lichtgealterter Haut und zur Aufhellung von unerwünschter Pigmentierung der Haut.

Gegenstand der Erfindung ist also auch die Verwendung der vorstehenden Zubereitungen und Wirkstoffe für die beschriebenen Zwecke, vorzugsweise aber die Verwendung zur Prophylaxe und Behandlung der folgenden Erscheinungen a) bis f), insbesondere der Dermatoheliosis:
a) Rückbildung des mikrovasculären Systems.
b) Schlaffheit und Ausbildung von Falten teilweise aufgrund einer Abnahme und Quervernetzung des Kollagens, Akkumulation der Glucosaminoglycane (Grundsubstanz).
c) Abflachung der Retezapfen, Damit verbunden ist die Verringerung der Fläche zwischen Dermis und Epidermis, über die Stoffe zur Ernährung und Entschlackung der Epidermis ausgetauscht werden.
d) Eingeschränkter regenerativer Turnover in der Epidermis, verbunden mit fehlerhafter Ausbildung der Hornschicht (Verhornungsstörungen), die zur Hautaustrocknung führt.
e) Fehlerhafte Regulation von Zellteilung (Profileration) und Zellreifung (Differenzierung) in der Epidermis, woraus zelluläre Atypen und der Verlust der Polarität resultieren.
f) Lokaler Hyper-, Hypo- und Fehlpigmentierungen (Altersflecken).

Die Bezeichnung Vitamin A-Säure und deren Derivate umfaßt all-trans-Retinsäure, auch Tretinoin oder Retinoesäure genannt, dessen Isomere, z.B. 13-cis-Retinsäure oder Isotretinoin und jeweils deren Derivate. Unter Derivaten sind z.B. auch Verbindungen zu verstehen, in denen die Carbonsäuregruppen mit Alkoholen verestert sind. Unter Isomeren der all-trans Retinsäure sind Verbindungen zu verstehen, in denen eine oder mehrere, z. B. bis zu drei Doppelbindungen statt in trans-Konfiguration in cis-Konfiguration vorliegen.

Geeignete Derivate der erfindungsgemäßen Vitamin A-Säuren auf deren Derivaten sind insbesondere die Salze. Auch Ester der Carbonsäuregruppen dieser erfindungsgemäßen Verbindungen mit Alkoholen sind bevorzugt.

Bevorzugte Salze sind wasserlösliche Salze, z.B. Natrium-, Kalium- und Ammoniumsalze oder Calciumsalze.

Geeignete Ester sind z.B. solche, die mit kurzkettigen oder mittelkettigen Alkanolen gebildet werden, vorzugsweise mit mono-Alkoholen. Sie können geradkettig oder verzweigt sein und z.B. 1 bis 12, vorzugsweise 1 bis 6 Kohlenstoffatome besitzen Bevorzugt werden Methanol, Ethanol, n-Propanol und iso-Propanol.

Ihre Alkylreste können auch einfach oder mehrfach ungesättigt sein und z.B. eine, zwei oder drei Doppelbindungen besitzen.

Weitere geeignete Alkohole mit denen erfindungsgemäße Ester gebildet werden, sind Retinole und alpha-Tocopherole mit denen z.B. Retinolretinoat oder DL-alpha-Tocopheryl-retinoat (Tocoretinat) erhalten wird.

Bevorzugte Verbindungen der Gruppe (B) sind Ubichinone und deren Derivate.

Besonders bevorzugt werden Zubereitungen mit einem Gehalt an all-trans-Retinsäure und Coenzym Q₁₀.

Ubichinone (auch Coenzyme Qₙ) sind eine Gruppe von Substanzen, die an ihrem Chinonring n Isopren-Einheiten kettenförmig gebunden haben (Q₀ - Q₁₀) Ubichinone fungieren bei der biologischen, mitochondralen Oxidation als Elektronenübertäger und spielen so eine bedeutende Rolle beim Energiestoffechsel der Zellen. Plastochinone sind analoge Verbindungen aus dem Pflanzenreich, die bei der Photosynthese eine Rolle spielen. Besonders bevorzugt werden Coenzym Q₉ und Coenzym Q₁₀.

Ubichinone (auch Coenzyme Qₙ) sind eine Gruppe von Substanzen, die an ihrem Chinonring n Isopren-Einheiten kettenförmig gebunden haben (Q₀ - Q₁₀). Ubichinone fungieren bei der biologischen, mitochondralen Oxidation als Elektronenüberträger und spielen so eine bedeutende Rolle beim Energiestoffwechsel der Zellen. Plastochinone sind analoge Verbinden aus dem Pflanzenbereich, die bei der Photosynthese eine Rolle spielen. In kosmetischen Zubereitungen finden Ubichinone seit langem Anwendung als Antioxidantien zum Schutz oxidationsempfindlicher Substanzen vor Sauerstoff-Radikal-induziertem Zerfall.

Mit "Ubichinone" und "Plastochinone" sind hier auch "Ubichinone und deren Derivate" und "Plastochinone und deren Derivate" gemeint.

Die Ubichinone sind aus der Literatur bekannt (z.B. "Römpp Chemie Lexikon, Georg Thieme Verlag, Stuttgart, New York, 9. Auflage, S. 4784-4785, oder "The Merck Index", 11th Edition, Merck & Co., Inc. Rahway, N.Y., USA, Abstr. 9751 (1989)). Sie werden auch als Mitochinone oder Coenzyme Q bezeichnet. Die Anzahl der Isopren-Einheiten in der Seitenkette wird mit n in der Bezeichnung Coenzyme Q-n angegeben, worin n eine ganze Zahl bedeutet. Bevorzugt werden Ubichinone oder Coenzyme Q-n mit n=0-12, besonders bevorzugt n=1-12 und insbesondere n=6 bis 10. Gegenstand der Erfindung ist also auch der Chinongrundkörper des Ubichinons ohne Isoprensubstituenten.

Erfindungsgemäße Ubichinone oder deren Derivate sind z.B. auch Alkyl-Ubichinone, insbesondere 6-Alkyl-Ubichinone mit vorzugsweise C₁-C₁₂-Alkyl-Resten. Bevorzugt wird Decyl-Ubichinon, insbesondere 6-Decyl-Ubichinon oder 2,3-Dimethoxy-5-methyl-6-decyl-1,4-benzochinon.

Die Plastochinone sind ebenfalls aus der Literatur bekannt (.B. "Römpp Chemie Lexikon", Georg Thieme Verlag, Stuttgart, New York, 9. Auflage, S. 3477). Sie sind in der Struktur eng mit den Ubichinonen verwandt und zählen auch zu den Isoprenoid-Chiononen, da sie am Chinonring eine Seitenkette aus Isopren-Einheiten tragen. Bevorzugt werden Plastochinone mit 0 - 12, besonders bevorzugt 1 - 10 und insbesondere 6 bis 10 Isopren-Einheiten in der Seitenkette. Gegenstand der Erfindung ist also auch der Chinongrundkörper des Plastochinons ohne Isoprensubstituenten.

Erfindungsgemäße Plastochinone oder deren Derivate sind z.B. auch Alkyl-Plastochinone mit vorzugsweise C₁-C₁₂-Alkyl-Resten. Bevorzugt werden Decyl-Plastochinone, insbesondere 5- oder 6-Decyl-Plastochinon oder 2,3-Dimethyl-5-decyl-1,4-benzochinon.

Ubichinone fungieren bei der biologischen, mitochondralen Oxidation als Elektronenüberträger und spielen so eine bedeutende Rolle beim Energiestoffwechsel der tierischen Zellen. In kosmetischen Zubereitungen finden Ubichinone seit langem Anwendung als Antioxidantien zum Schutz oxidationsempfindlicher Substanzen.

Plastochinone sind analoge Verbindungen aus dem Pflanzenbereich, die bei der Photosynthese in den Chloroplasten der pflanzlichen Zellen eine Rolle spielen. Sie unterscheiden sich von den Ubichinonen in drei Substituenten am Chinonring, wobei die zwei Methoxy-Gruppen in den Ubichinonen durch Methylgruppen und eine Methylgruppe durch ein Wasserstoffatom ersetzt sind. Strukturgleich sind jedoch die kettenförmig gebundenen Isopren-Einheiten (vergl. z.B. Pfister und Arntzen, Z. für Naturfoschung C34; 996ff, 1979).

Besonders bevorzugt werden die folgenden erfindungsgemäßen Wirkstoffe und Kombinationen damit:
Coenzym Q - 10, Coenzym Q - 9, Coenzym Q - 8, Coenzym Q - 7, Coenzym Q - 6,
Plastochinon mit 10 Isopreneinheiten (auch PQ-10 genannt entsprechend der IUB-Abkürzung PQ für Plastochinone, in der Formel PQ-n soll n die Anzahl der Isopren-Einheiten (0 bis 12) angeben), PQ-9, PQ-8, PQ-7, PQ-6,

### Vitamin A-Säure (Tretinoin).

Es hat sich überraschenderweise gezeigt, daß Vitamin A-Säure oder deren Derivate in Kombination mit Ubichinonen und/oder Plastochinonen bei der Akne-Prophylaxe bzw. beim Schutz vor Lichtalterung bzw. bei der Reparatur von lichtbedingten Strukturschäden der Haut in synergistischer Weise zusammenwirken, was in signifikanter Weise dem Nachteil des Standes der Technik abhilft. Die erfindungsgemäße Wirkstoffkombination und topische Zubereitungen damit sind auch hervorragend zur Aufhellung unerwünschter Pigmentierung der Haut geeignet und zwar auch prophylaktisch.

Besonders vorteilhaft ist, daß in den erfindungsgemäßen Kombinationen die üblicherweise in topischen Zubereitungen angewendeten Mengen von Vitamin A-Säure gesenkt werden kann ohne daß eine Wirkungsminderung eintritt. Außerdem wird durch die Komponenten (B) die Stabilität der Vitamin A-Säure in den Zubereitungen erhöht und eine längere Lagerung möglich.

Unter Pigmentierung (bzw. pigmentiert) werden alle Verfärbungen der (bzw. verfärbte) Haut verstanden.

Zu der Prävention oder auch Prophylaxe und Behandlung unerwünschter Pigmentierung der Haut, zählen beispielsweise die Aufhellung dunkler Haut, aber auch die Aufhellung oder Entfernung von Hyperpigmentierungen, insbesondere lokalen Hyperpigmentierungen, und Fehlpigmentierungen, die beispielsweise auch unter dem Begriff "skin-whitening (Effekt)" bekannt sind und auch die vorbeugenden Anwendungen zu diesen Zwecken.

Weiterhin zählt zu der Prävention oder Prophylaxe und Behandlung unerwünschter Pigmentierung der Haut die Verhinderung und Behandlung der Hautbräunung, insbesondere der durch UV-Strahlung hervorgerufenen Hautbräunung.

Geeignete Konzentrationen von Vitamin A-Säuren oder deren Derivaten im Sinne der Erfindung in topischen Zubereitungen liegen vorzugsweise zwischen 0.0001 und 10 Gew.-%. Die wirksamen Konzentrationen von Ubichinonen und/oder Plastochinonen in topischen Zubereitungen liegen vorzugsweise zwischen 0.001 und 90 Gew.-%.

Vorteilhaft enthalten erfindungsgemäße Hautpflegeprodukte oder Dermatika Kombinationen in folgender Zusammenstellung:
0,0001 - 1 Gew.-% Vitamin A-Säure oder deren Derivate
0,001 - 10 Gew.- % Ubichinon und/oder Plastochinon.

Bevorzugt enthalten Hautpflegepräparate oder Dermatika
0,001 - 1 Gew.-% Vitamin A-Säure oder deren Derivate und
0,01 -1 Gew.-% Ubichinon und/oder Plastochinon, insbesondere

0,001 - 1 Gew.-% all-trans Retinsäure und
0,01 - 1 Gew.% Coenzym Q₁₀.

Ganz besonders bevorzugt enthalten die Hautpflegeprodukte oder Dermatika
0,01 Gew.-% all-trans Retinsäure
0,3 Gew.-% Coenzym Q₁₀.

Im Rahmen der Anmeldung sind Gewichtsprozente bezogen auf 100% Gesamtzusammensetzung des jeweiligen erfindungsgemäßen Hauptpflegepräparates oder Dermatikums gemeint.

Die erfindungsgemäßen Wirkstoffkombinationen oder Wirkstoffe können in den topischen Zubereitungen in Mengen von 0,0001 bis 99 Gew.-%, z.B. auch in Mengen von 0,001 bis 50 Gew.-% vorliegen, jeweils bezogen auf das Gesamtgewicht der Zubereitungen.

Vorzugsweise können die erfindungsgemäßen Wirkstoffkombinationen oder Wirkstoffe in den topischen Zubereitungen in Mengen von 0,01 bis 10 Gew.-%, insbesondere in Mengen von 0,1 bis 1 Gew.-% vorliegen, jeweils bezogen auf das Gesamtgewicht der Zubereitungen.

In den Kombinationen können die Gewichtsverhältnisse (A) : (B) der beiden Komponenten in großen Bereichen schwanken, z.B. im Verhältnis 1 : 100 bis 1 : 1, vorzugsweise im Verhältnis 1 : 15 bis 1 : 1. Sie können z.B. auch im Gewichtsverhältnis 1: 2 bis 1 : 1 oder 1 : 1 vorliegen.

Erfindungsgemäße topische Zubereitungen oder Zusammensetzungen mit den erfindungsgemäßen Kombinationen und Wirkstoffen sind alle gängigen Anwendungsformen, z.B. Cremes (W/O, O/W, W/O/W), Gele, Lotionen, Milchen.

Die erfindungsgemäßen topischen Zubereitungen können als flüssige, pastöse oder feste Zubereitungen formuliert werden, beispielsweise als wäßrige oder alkoholische Lösungen, wäßrige Suspensionen, Emulsionen, Salben, Cremes, Öle, Pulver oder Stifte. In Abhängigkeit von der gewünschten Formulierung können Wirkstoffe in pharmazeutische und kosmetische Grundlagen für topische Applikationen eingearbeitet werden, die als weitere Komponenten beispielsweise Ölkomponenten, Fett und Wachse, Emulgatoren, anionische, kationische, ampholytische, zwitterionische und/oder nichtionogene Tenside, niedere ein- und mehrwertige Alkohole, Wasser, Konservierungsmittel, Puffersubstanzen, Verdickungsmittel, Duftstoffe, Farbstoffe und Trübungsmittel enthalten. Vorteilhaft können die erfindungsgemäßen Wirkstoffe auch in transdermalen therapeutischen Systemen, insbesondere kubischen Systemen verwendet werden.

Es ist ferner von Vorteil, den Zubereitungen Antioxidantien (z.B. alpha-Tocopherol, Vitamin A und C, Imidazole, alpha-Hydroxycarbonsäuren (z.B. Äpfelsäure, Glycolsäure, Gluconsäure, Salicylsäure sowie deren Derivate) undloder Eisenkomplexbildner (z.B. EDTA, alpha-Hydoxyfettsäuren) und/oder bekannte UV-Lichtschutzfilter in Mengen von z.B. 0,1 bis 10 Gew.-% zuzusetzen, um die Stabilität der oxidationsempfindlichen Wirkstoffe zu gewährleisten.

Auch ist es vorteilhaft, den Zubereitungen insbesondere 0,01 - 10 Gew.-% an Stoffen bzw. Stoffkombinationen des aeroben zellulären Energiestoffwechsels (z.B. zelluläre Energieüberträger wie Kreatin, Guanin, Guanosin, Adenin, Nicotin, Nicotinamid, Riboflavin), Coenzyme (z.B. Pantothensäure, Panthenol, Liponsäure, Niacin), Hilfsfaktoren (z.B. L-Carnitin, Uridin), Substrate (z.B. Hexosen, Pentosen, Fettsäuren) und intermediäre Stoffwechselprodukte (z.B. Zitronensäure, Pyruvat) und/oder Glutathion zuzusetzen.

Vorteilhaft können erfindunsgemäße Zubereitungen außerdem Substanzen enthalten, die UV-Strahlung im UVA- und/oder im UVB-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1,0 bis 6,0 Gew.-% beträgt, bezogen auf das Gesamtgewicht der Zubereitungen, um kosmetische Zubereitungen zur Verfügung zu stellen, die die Haut vor dem gesamten Bereich der ultravioletten Strahlung schützen. Sie können auch als Sonnenschutzmittel für die Haut dienen. In den Zubereitungen wirken die UV-Absorber gegenüber den Wirkstoffen als Antioxidantien.

Enthalten die erfindungsgemäßen Emulsionen UVB-Filtersubstanzen, können diese öllöslich oder wasserlöslich sein. Erfindungsgemäß vorteilhafte öllösliche UVB-Filter sind z.B.:
3-Benzylidencampher-Derivate, vorzugsweise 3-(4-Methylbenzyliden)campher, 3-Benzylidencampher.

### Vorteilhaft wasserlösliche UVB-Filter sind z.B.:

Salze der 2-Phenylbenzimidazol-5-sulfonsäure wie ihr Natrium-, Kalium- oder ihr Triethanolammonium-Salz. sowie die Sulfonsäure selbst.

Es kann auch von Vorteil sein, erfindungsgemäße Wirkstoffkombinationen mit UVA-Filtern zu kombinieren, die bisher üblicherweise in kosmetischen Zubereitungen enthalten sind. Bei diesen Substanzen handelt es sich vorzugsweise um Derivate des Dibenzoylmethans, insbesondere um 1-(4'-tert.Butylpheny)-3(4'-methoxyphenyl)propan-1,3-dion und um 1-Phenyl-3-(4'-lsopropylphenyl)propan-1,3dion. Auch diese Kombinationen bzw. Zubereitungen, die diese Kombinationen enthalten, sind Gegenstand der Erfindung. Es können die für die UVB-Kombination verwendeten Mengen eingesetzt werden.

Gegenstand der Erfindung sind also auch die Kombinationen der erfindungsgemäßen Wirkstoffe, insbesondere in den topischen Zubereitungen, mit Antioxidantien, Stoffen des aeroben zellulären Energiestoffwechsels und/oder UV-Absorbern, durch die sich z.B. die Stabilität und die Wirkung der Zubereitung verbessern läßt.

Darüber hinaus können schützende Formulierungsformen angewendet werden, wobei die erfindungsgemäßen Stoffe z.B. in Liposomen, Micellen, Nanosphären usw. aus z.B. hydrierten Amphiphilen, wie z.B. Ceramiden, Fettsäuren, Sphingomyelin und Phosphoglyceriden bzw. in Zyklodextrane eingeschlossen (verkapselt) werden. Weiterer Schutz kann durch die Verwendung von Schutzgas (z.B. N₂, CO₂) bei der Formulierung und die Verwendung gasdichter Verpackungsformen erreicht werden.

Weitere Hilfs- und Zusatzstoffe können wasserbindende Stoffe, Verdicker, Füllstoffe, Parfüm, Farbstoffe, Emulgatoren, Wirkstoffe wie Vitamine, Konservierungsmittel, Wasser und/oder Salze sein.

Bei der Verarbeitung der Wirkstoffe und anderer oxidationsempfindlicher Stoffe sollte die Temperatur nicht über 40°C liegen. Ansonsten sind die üblichen Maßregeln zu beachten, die dem Fachmann bekannt sind.

Die erfindungsgemäßen Stoffgruppen lassen sich so in alle Grundlagen einarbeiten. Grundsätzlich sind allerdings W/O- und O/W- und W/O/W-Emulsionen bevorzugt. Besonders vorteilhaft können erfindungsgemäße Kombinationen in Produkte wie beispielsweise O/W-Cremes, W/O-Cremes, O/W-Lotionen oder W/O-Lotionen eingesetzt werden.

Mengenangaben, Prozentangaben oder Teile beziehen sich, soweit nicht anders angegeben, auf das Gewicht, insbesondere auf das Gesamtgewicht der Zubereitungen oder der jeweiligen Mischung.

Die folgenden Beispiele dienen dazu, die Erfindung zu beschreiben. Die Mengenangaben sind Gewichtsteile oder Gewichtsprozent bei den "100" Angaben.

### Beispiel I

### Hautcreme vom W/O-Typ

| | |
|---|---|
| Vaseline DAB 9 | 13 |
| Glycerin DAB 9 | 6,3 |
| Wasser VES | 34,4 |
| Paraffinöl (Mineralöl 5E,Shell) | 31 |
| Cetearylalkohol/TEG-40-Castor Oil/Natriumcetearylsulfat (Eutanol G, Henkel KGaA) | 2,5 |

In die 75°C warme Fettphase werden 0,3 Teile Coenzym Q₁₀ in 3 Teilen Paraffinöl gelöst eingearbeitet. Die Fettphase wir sodann zu der 75°C warmen Wasserphase gegeben, verrührt und homogenisiert, bis eine gleichmäßige hellgelbe Creme entstanden ist. 0,03 Teile all-trans Retinsäure werden bei Raumtemperatur in weiteren 3,47 Teilen Paraffinöl gelöst und in die abgekühlte Creme eingerührt.

### Beispiel I hat folgende endgültige Zusammensetzung:

| | |
|---|---|
| Vaseline DAB 9 | 13 |
| Glycerin DAB 9 | 6,3 |
| Wasser VES | 34,4 |
| Paraffinöl | |
| (Mineralöl 5E,Shell) | 43,47 |
| Cetearylalkohol/TEG-40-Castor Oil/Natriumcetearylsulfat (Eutanol G, Henkel KGaA) | 2,5 |
| all-trans-Retinsäure | 0,03 |
| Coenzym Q₁₀ | 0,3 |
| | 100 |

### Beispiel II

### Hautcreme vom W/O-Typ

| | |
|---|---|
| PEG-1 Glyceryl-Oleostearat + Paraffinwachs | 8 |
| Vaseline DAB | 2,8 |
| Paraffinwachs/Paraffin | 1,8 |
| Ceresin | 2,2 |
| Octyldodecanol | 10 |
| Propylenglycol | 1 |
| Glycerin | 1 |
| Magnesiumsulfat | 0,7 |
| Wasser VES | 59,7 |
| Summe Additive (Parfüm, Konservierung, Stabilisation) | 0,8 |

In die 75°C warme Fettphase werden 0,2 Teile Coenzym Q₁₀ und 0,2 Teile Coenzym Q₆ in 6 Teilen Paraffinöl gelöst eingearbeitet. Die Fettphase wir sodann zu der 75°C warmen Wasserphase gegeben, verrührt und homogenisiert, bis eine gleichmäßige hellgelbe Creme entstanden ist. 0,02 Teile all-trans Retinsäure werden bei Raumtemperatur in weiteren 5,88 Teilen Paraffinöl gelöst und in die abgekühlte Creme eingerührt.

Beispiel II hat folgende endgültige Zusammensetzung:

| | |
|---|---|
| PEG-1 Glyceryl-Oleostearat + Paraffinwachs | 8 |
| Vaseline DAB | 2,8 |
| Paraffinwachs/Paraffin | 1,8 |
| Paraffinöl (Mineralöl 5E,Shell) | 11,88 |
| Ceresin | 2,2 |
| Octyldodecanol | 10 |
| all-trans-Retinsäure | 0,02 |
| Coenzym Q₆ | 0,2 |
| Coenzym Q10 | 0,2 |
| Propylenglycol | 1 |
| Glycerin | 1 |
| Magnesiumsulfat | 0,7 |
| Wasser VES | 59,4 |
| Summe Additive (Parfüm, Konservierung, Stabilisation) | 0,8 |
| | 100 |

### Beispiel III

### Hautcreme vom O/W-Typ

| | |
|---|---|
| Octyldodecanol (Emulgade F, Henkel KGaA) | 9,3 |
| Cetearylalkohol/TEG-40-Castor Oil/Natriumcetearyl- | |
| sulfat (Eutanol G, Henkel KGaA) | 3,7 |
| Wasser VES | 73,7 |
| Glycerin DAB 9 | 4,6 |

In die 75°C warme Fettphase werden 0,9 Teile Coenzym Q₁₀ in 4 Teilen Paraffinöl gelöst eingearbeitet. Die Fettphase wir sodann zu der 75°C warmen Wasserphase gegeben, verrührt und homogenisiert, bis eine gleichmäßige hellgelbe Creme entstanden ist. 0,01 Teile all--trans Retinsäure werden bei Raumtemperatur in weiteren 3,79 Teilen Paraffinöl gelöst und in die abgekühlte Creme eingerührt.

### Beispiel III hat folgende endgültige Zusammensetzung:

| | |
|---|---|
| Octyldodecanol (Emulgade F, Henkel KGaA) | 9,3 |
| Cetearylalkohol/TEG-40-Castor Oil/Natriumcetearylsulfat (Eutanol G, Henkel KGaA) | 3,7 |
| Wasser VES | 73,7 |
| Glycerin DAB 9 | 4,6 |
| Paraffinöl | |
| (Mineralöl eE, Shell) | 7,79 |
| Coenzym Q₁₀ | 0,9 |
| all-trans-Retinsäure | 0.01 |
| | 100 |

### Beispiel IV

### O/W-Lotion

| | |
|---|---|
| Steareth-2 | 3 |
| Steareth-21 | 2 |
| Cetearylalkohol/TEG-40-Castor Oil/Natriumcetearylsulfat (Eutanol G, Henkel KGaA) | 2,5 |
| Propylenglycol | 1 |
| Glycerin | 1 |
| Wasser VES | 74,3 |
| Summe Additive (Parfüm, Konservierung, Stabilisation) | 0,8 |

In die 75°C warme Fettphase werden 0,1 Teile Coenzym Q₁₀ in 5,2 Teile Paraffinöl gelöst eingearbeitet. Die Fettphase wir sodann zu der 75°C warmen Wasserphase gegeben, verrührt und homogenisiert, bis eine gleichmäßige hellgelbe Lotion entstanden ist. 0,05 Teile all-trans Retinsäure werden bei Raumtemperatur in weiteren 9,85 Teilen Paraffinöl gelöst und in die abgekühlte Lotion eingerührt.

Beispiel IV hat folgende endgültige Zusammensetzung:

| | |
|---|---|
| Steareth-2 | 3 |
| Steareth-21 | 2 |
| Cetearylalkohol/TEG-40-Castor Oil/Natriumcetearylsulfat (Eutanol G, Henkel KGaA) | 2,5 |
| Paraffinöl (Mineralöl eE, Shell) | 14,85 |
| Propylenglycol | 1 |
| Coenzym Q₁₀ | 0,1 |
| all-trans-Retinsäure | 0,05 |
| Glycerin | 1 |
| Wasser VES | 74,3 |
| Summe Additive (Parfüm, Konservierung, Stabilisation) | 0,8 |
| | 100 |

### Beispiel V

### O/W-Lotion

| | |
|---|---|
| Octyldodecanol (Emulgade F, Henkel KGaA) | 5,6 |
| Cetearylalkohol/TEG-40-Castor Oil/Natriumcetearylsulfat (Eutanol G, Henkel KGaA) | 8,9 |
| Cetearylisononanoat (Cetiol 5N, Henkel KGaA) | 7,5 |
| Wasser VES | 62,3 |
| Glycerin DAB 9 | 4,7 |

In die 75°C warme Fettphase werden 0,4 Teile Coenzym Q₁₀ in 6 Teilen Paraffinöl gelöst eingearbeitet. Die Fettphase wir sodann zu der 75°C warmen Wasserphase gegeben, verrührt und homogenisiert, bis eine gleichmäßige hellgelbe Lotion entstanden ist. 0,04 Teile all-trans Retinsäure werden bei Raumtemperatur in weiteren 4,56 Teilen Paraffinöl gelöst und in die abgekühlte Lotion eingerührt.

Beispiel V hat folgende endgültige Zusammensetzung:

| | |
|---|---|
| Octyldodecanol (Emulgade F, Henkel KGaA) | 5,6 |
| Cetearylalkohol/TEG-40-Castor Oil/Natriumcetearylsulfat (Eutanol G, Henkel KGaA) | 8,9 |
| Cetearylisononanoat (Cetiol 5N, Henkel KGaA) | 7,5 |
| Wasser VES | 62,3 |
| Glycerin DAB 9 | 4,7 |
| Paraffinöl (Mineralöl eE, Shell) | 10,56 |
| Coenzym Q₁₀ | 0,4 |
| all-trans-Retinsäure | 0,04 |
| | 100 |

### Beispiel VI

### Hautöl

| | |
|---|---|
| Glyceryltricaprylat (Miglyol 812, Dynamit Nobel) | 21 |
| Hexyllaurat (Cetiol A, Henkel KGaA) | 20 |
| Octylstearat (Cetiol 886, Henkel KGaA | 20 |
| Paraffinöl (Mineralöl 5E, Shell) | 36,98 |
| all-trans-Retinsäure | 0,02 |
| Coenzym Q₆ | 1,6 |
| Coenzym Q₁₀ | 0,4 |
| | 100 |

Die Komponenten werden bei 25°C verrührt, bis eine gleichmäßige, klare Mischung entstanden ist.

### Beispiel VII

### Hautöl

| | |
|---|---|
| Glyceryltricaprylat (Miglyol 812, Dynamit Nobel) | 21 |
| Hexyllaurat (Cetiol A, Henkel KGaA) | 20 |
| Octylstearat (Cetiol 886, Henkel KGaA | 20 |
| Paraffinöl (Mineralöl 5E, Shell) | 36,98 |
| Tocoretinat | 0,02 |
| Coenzym Q10 | 2,0 |
| | 100 |

## Patentansprüche

1. Topische Zubereitungen mit einem Gehalt an einer Verbindung oder mehreren Verbindungen, ausgewählt aus der Gruppe (A), bestehend aus Vitamin A-Säure oder deren Derivaten, in Kombination mit einem Gehalt an einer Verbindung oder mehreren Verbindungen, ausgewählt aus der Gruppe (B), bestehend aus Ubichinonen und deren Derivaten und Plastochinonen und deren Derivaten.

2. Zubereitungen gemäß Anspruch 1 mit einem Gehalt an einer Verbindung oder zwei Verbindungen oder drei Verbindungen, ausgewählt aus der Gruppe (A), kombiniert mit einer Verbindung oder zwei Verbindungen oder drei Verbindungen aus der Gruppe (B).

3. Zubereitungen gemäß Anspruch 1, **dadurch gekennzeichnet, daß** sie all-trans Retinsäure enthalten.

4. Zubereitungen gemäß Anspruch 1-3, **dadurch gekennzeichnet, daß** sie mindestens ein Ubichinon enthalten.

5. Zubereitungen gemäß Anspruch 1, **dadurch gekennzeichnet, daß** sie all-trans-Retinsäure und Coenzym Q-10 enthalten.

6. Zubereitungen gemäß Anspruch 1, **dadurch gekennzeichnet, daß** sie Ubichinone oder Plastochinone mit 0 bis 12 Isopreneinheiten und/oder gegebenenfalls Alkyl-Reste besitzen.

7. Zubereitungen gemäß Anspruch 1, **dadurch gekennzeichnet, daß** die Ubichinone oder Plastochinone 9 oder 10 Isopreneinheiten besitzen.

8. Zubereitungen gemäß Anspruch 1, **dadurch gekennzeichnet, daß** sie Antioxidantien, Stoffe des aeroben zellulären Energiestoffwechsels und/oder UV-Absorber enthalten.

9. Zubereitungen gemäß Anspruch 1, **dadurch gekennzeichnet, daß** sie kosmetische oder dermatologische Zubereitungen, insbesondere W/O-, O/W- oder W/O/W-Emulsionen sind.

10. Verwendung einer Verbindung oder mehreren Verbindungen ausgewählt aus der Gruppe (A), bestehend aus Vitamin A-Säure oder deren Derivaten, in Kombination mit einem Gehalt an einer Verbindung oder mehreren Verbindungen, ausgewählt aus der Gruppe (B), bestehend aus Ubichinonen und deren Derivaten und Plastochinonen und deren Derivaten, zur Herstellung topischer Zubereitungen zur Pflege und prophylaktischen Behandlung bei Akne und zur Pflege und Prophylaxe bei Lichtalterung und zur Behandlung lichtgealterter Haut und zur Aufhellung von unerwünschter Pigmentierung der Haut.

## Claims

1. Topical preparations containing one or more compounds chosen from group (A), which consists of vitamin A acid or its derivatives, in combination with one or more compounds chosen from group (B), which consists of ubiquinones and their derivatives and plastoquinones and their derivatives.

2. Preparations according to Claim 1 containing one, two or three compounds chosen from group (A), combined with one, two or three compounds from group (B).

3. Preparations according to Claim 1, **characterized in that** they contain all-trans retinoic acid.

4. Preparations according to Claims 1 - 3, **characterized in that** they contain at least one ubiquinone.

5. Preparations according to Claim 1, **characterized in that** they contain all-trans retinoic acid and coenzyme Q₁₀.

6. Preparations according to Claim 1, **characterized in that** they possess ubiquinones or plastoquinones with 0 - 12 isoprene units and/or possibly alkyl residues.

7. Preparations according to Claim 1, **characterized in that** the ubiquinones or plastoquinones possess 9 or 10 isoprene units.

8. Preparations according to Claim 1, **characterized in that** they contain antioxidants, substances of aerobic cellular energy metabolism and/or UV absorbers.

9. Preparations according to Claim 1, **characterized in that** they are cosmetic or dermatological preparations, especially W/O, O/W or W/O/W emulsions.

10. Use of one or more compounds chosen from group (A), which consists of vitamin A acid or its derivatives, in combination with one or more compounds chosen from group (B), which consists of ubiquinones and their derivatives and plastoquinones and their derivatives, for the production of topical preparations for the care and prophylactic treatment of acne and for the care and prophylaxis of light-aging and for the treatment of light-aged skin and for the lightening of undesired pigmentation of the skin.

## Revendications

1. Compositions topiques contenant un composé ou plusieurs composés, choisi(s) dans le groupe (A) constitué de l'acide de vitamine A ou de ses dérivés, en combinaison avec un composé ou plusieurs composés choisi(s) dans le groupe (B) constitué des ubiquinones et de leurs dérivés et des plastoquinones et de leurs dérivés.

2. Compositions selon la revendication 1, contenant un composé ou deux composés ou trois composés choisis dans. le groupe (A), combinée à un composé ou deux composés ou trois composés du groupe (B).

3. Compositions selon la revendication 1, **caractérisées en ce qu'**elles contiennent de l'acide rétinique tout-trans.

4. Compositions selon la revendication 1 à 3, **caractérisées en ce qu'**elles contiennent au moins une ubiquinone.

5. Compositions selon la revendication 1, **caractérisées en ce qu'**elles contiennent de l'acide rétinique tout-trans et de la coenzyme Q10.

6. Compositions selon la revendication 1, **caractérisées en ce qu'**elles contiennent des ubiquinones ou des plastoquinones, dotés de 0 à 12 unités isoprène et/ou le cas échéant de radicaux alkyle.

7. Compositions selon la revendication 1, **caractérisées en ce que** les ubiquinones ou les plastoquinones possèdent de 9 à 10 unités isoprène.

8. Compositions selon la revendication 1, **caractérisées en ce qu'**elles contiennent des antioxydants, des substances de régénération énergétique du tissu cellulaire aérobie et/ou des absorbeurs d'UV.

9. Compositions selon la revendication 1, **caractérisées en ce que** ce sont des compositions cosmétiques ou dermatologiques, en particulier des émulsions E/H, H/E ou E/H/E.

10. Utilisation d'un composé ou de plusieurs composés choisis parmi le groupe (A), constitué d'acide de vitamine A ou de ses dérivés, en combinaison avec une teneur en un composé ou plusieurs composés choisis parmi le groupe (B), constitué des ubiquinones et de leurs dérivés et des plastoquinones et de leurs dérivés, pour fabriquer des compositions topiques de soins pour le traitement prophylactique de l'acné, de soins et de prophylaxie du vieillissement de la peau provoqué par les UV, de traitement de la peau ayant subi un vieillissement provoqué par les UV et d'éclaircissement de la pigmentation indésirable de la peau.
